**Europäisches Patentamt**

(19) **European Patent Office**

**Office européen des brevets**

(11) Veröffentlichungsnummer: **0 164 037 B1**

# (12) EUROPÄISCHE PATENTSCHRIFT

(45) Veröffentlichungstag der Patentschrift:
**16.08.89**

(21) Anmeldenummer: **85106423.8**

(22) Anmeldetag: **24.05.85**

(51) Int. Cl.⁴: **G 01 N 21/47, C 12 M 1/34, G 02 B 5/02**

(54) **Beleuchtungseinrichtung für die optische, insbesondere bildanalytische Auswertung von mikrobiologischen Objekten.**

(30) Priorität: **04.06.84 DE 3420760**

(43) Veröffentlichungstag der Anmeldung:
**11.12.85 Patentblatt 85/50**

(45) Bekanntmachung des Hinweises auf die Patenterteilung:
**16.08.89 Patentblatt 89/33**

(84) Benannte Vertragsstaaten:
**AT BE CH DE FR GB IT LI NL SE**

(56) Entgegenhaltungen:
**EP-A- 0 065 409**
**DE-A- 2 847 011**
**US-A- 4 448 534**

(73) Patentinhaber: **BAYER AG,**
**D-5090 Leverkusen 1 Bayerwerk (DE)**

(72) Erfinder: **Dedden, Hubert, Dip.-Ing., Salmstrasse 27,**
**D-4018 Langenfeld (DE)**
Erfinder: **Förster, Dietrich, Dr., Fuchsbergerstrasse 51,**
**D-4010 Hilden (DE)**
Erfinder: **Rost, Horst, Dr., Ehrenmalstrasse 21,**
**D-4130 Moers (DE)**
Erfinder: **Zembrod, Alfred, Dr., Nittumerweg 60,**
**D-5060 Bergisch-Gladbach 2 (DE)**

## Beschreibung

Die Erfindung betrifft eine Beleuchtungseinrichtung für die optische, insbesondere bildanalytische Auswertung einer mikrobiologischen Struktur. Die Beleuchtungseinrichtung umfasst eine diffus strahlende, flächenhafte Lichtquelle und ein zwischen der mikrobiologischen Struktur und der Lichtquelle angeordnetes Blendensystem. Instrumente zur Untersuchung des Bakterienwachstums auf geeigneten Nährböden sind heute ein unentbehrliches Hilfsmittel der mikrobiologischen Forschung. Die Nährböden werden dabei entweder an vorgegebenen Positionen oder flächenhaft verteilt angeimpft. Es entstehen dann charakteristische Wachstumsmuster (im folgenden als mikrobiologische Struktur bezeichnet), die entweder eine periodische oder eine flächenhafte Objektstruktur besitzen. Der Charakter, d.h. das makroskopische Erscheinungsbild und die für eine bildanalytische Auswertung der mikrobiologischen Struktur wichtigen optischen Eigenschaften (Absorption, Brechung, Streuung) hängen davon ab, welches Verfahren zur Untersuchung des Bakterienwachstums benutzt wird. Folgende mikrobiologische Standardverfahren haben sich eingebürgert:

1. Agardilution (AD)
2. Mikrodilution (MD)
3. Hemmhofmessung auf Petrischalen (HH-P)
4. Hemmhofmessung auf grossen rechteckigen Platten (HH-XY)
5. Koloniezählung (KZ)

Diese Verfahren sind in der Fachliteratur ausführlich beschrieben und brauchen daher hier nicht näher erläutert zu werden. Allen Verfahren gemeinsam ist das bildanalytische Problem, Ojektpositionen oder Flächenbereiche, in denen Bakterienwachstum auftritt, durch Abtastung mit einer Videokamera zu lokalisieren und/oder quantitativ auszuwerten. Inwieweit Bakterienwachstum auftritt, hängt ab von der Wirkung bestimmter Antibiotikamengen, die in die Nährböden eingearbeitet werden. Bei Anwendung des Agar- und Mikrodilutionsverfahrens kann Bakterienwachstum nur an festgelegten Positionen erfolgen. Dagegen findet bei der Methode der Hemmhofmessung ein flächenhaftes Bakterienwachstum statt. Bei der Methode Kolonienzählung tritt zwar ein punktförmiges Bakterienwachstum in Form von Einzelkolonien mit einem Durchmesser von wenigen Millimetern auf. Die Positionen der einzelnen Kolonien sind jedoch unregelmässig über die Nährbodenfläche verteilt.

Die bildanalytische Auswertung von mikrobiologischen Strukturen ist häufig mit Schwierigkeiten verbunden, weil die Strukturen oft sehr kontrastarm und in einer ungleichmässigen («unruhigen») Umgebung eingebettet sind, deren Helligkeitsverteilung weitgehend mit der Helligkeitsverteilung der Struktur übereinstimmt. In solchen Fällen kann man auch durch elektronische Mittel kaum noch Verbesserungen erzielen. Dagegen hat sich gezeigt, dass der Kontrast zwischen Objekt und Untergrund entscheidend von der Beleuchtungsart abhängt. In der Anfangsphase der mikrobiologischen Untersuchungsmethoden wurden noch optische Transmissionsmessungen durchgeführt. Moderne Apparaturen zur Untersuchung biologischer Vorlagen arbeiten in der Regel mit diesem Beleuchtungsprinzip. Nachdem sich experimentelle Anhaltspunkte ergeben hatten, dass sich die Kontrastverhältnisse durch eine verbesserte Beleuchtungseinrichtung aller Voraussicht nach noch steigern lassen, wurden Weiterentwicklungen in dieser Richtung durchgeführt und schliesslich eine neue Beleuchtungseinrichtung konzipiert. Dabei lag das Problem zugrunde, dass die verwendete ortsfeste Fernsehkamera alle in Frage kommenden mikrobiologischen Strukturen in einer Streulichtanordnung von oben her abtastet und bildmässig auswertet und die mikrobiologischen Strukturen von unten her durch schräg einfallendes Licht derart beleuchtet werden, dass ein geradliniger direkter Lichteinfall in die Kamera vermieden wird. Die Kamera soll also nur das an der mikrobiologischen Struktur gestreute Licht erfassen, während die von der Lichtquelle kommenden direkten Strahlen ausgeblendet werden. Dieses Prinzip sollte für alle vorkommenden Wachstumsmuster und Plattengrössen (bis zu Rechteckplatten der Grösse 30 × 30 cm) realisiert werden.

Diese Aufgabe wird, ausgehend von einer Beleuchtungseinrichtung mit einer diffus strahlenden, flächenhaften Lichtquelle und einem zwischen der mikrobiologischen Struktur und der Lichtquelle angeordneten Blendensystem erfindungsgemäss dadurch gelöst,

a) dass das Blendensystem durch zwei hintereinandergeschaltete, im Abstand befindliche, optisch komplementäre Blendenraster gebildet wird,

b) dass das erste Blendenraster aus einer Vielzahl von transparenten, periodisch aufeinanderfolgenden Kreisscheiben auf einem lichtundurchlässigen Untergrund, und das zweite Blendenraster aus einer Vielzahl von lichtundurchlässigen, periodisch aufeinanderfolgenden Kreisscheiben auf transparentem Untergrund besteht,

c) und dass die lichtundurchlässigen Kreisscheiben in ihrem Durchmesser grösser sind als die transparenten Kreisscheiben, so dass sich die Kreisscheiben der beiden Blendenraster in der Projektion überlappen.

Eine nach diesem Prinzip aufgebaute Beleuchtungseinrichtung führte bei allen mikrobiologischen Messverfahren zu einer Steigerung der Kontrastverhältnisse und damit zu einer erhöhten Sicherheit, Reproduzierbarkeit und Genauigkeit bei der bildanalytischen Auswertung.

Besteht die mikrobiologische Struktur aus einer Vielzahl von Einzelobjekten, wie dies z.B. bei dem Agar- und Mikrodilutionsverfahren der Fall ist, so kann durch folgende konstruktive Massnahmen eine weitere Verbesserung erzielt werden: Die beiden Blendenraster werden in der Weise hintereinander und unterhalb der mikrobiologischen Struktur angeordnet, dass die Mittelpunkte der Kreisscheiben der beiden Blendenraster auf den verlängerten Verbindungsgeraden von einem auf der optischen Achse liegenden Fluchtpunkt zu den Objektpositionen liegen. Der Fluchtpunkt stimmt im allgemeinen mit dem Mittelpunkt des Kameraobjektivs überein. Mit dieser Anordnung lässt sich eine optimale Ausblendung von direktem Lichteinfall in die Kamera erreichen, wenn die oben angegebene Bedingung für alle perspektivischen Winkel gilt, unter denen die Kamera die Objektpositionen sieht.

Im Gegensatz zum Agardilutions- und Mikrodilutionsverfahren besteht die zu untersuchende mikrobiologische Struktur beim Hemmhofverfahren aus relativ grossen flächenhaften Objekten. In diesem Fall werden die beiden Blendenraster vorteilhaft so hintereinandergeschaltet, dass die transparenten Kreisscheiben des ersten Blendenrasters im wesentlichen konzentrisch in Parallelprojektion zu den undurchlässigen Kreisscheiben des zweiten Blendenrasters angeordnet sind.

Für die optimale Geometrie der Blendenraster wurden folgende Bereiche ermittelt:

a) Der Abstand h zwischen den beiden Blendenrastern wird 0,5 bis 5 mal so gross gewählt, wie der Durchmesser der transparenten Kreisscheiben des ersten Blendenrasters.

b) Die Differenz $d_2-d_1$ der Durchmesser der beiden Kreisscheiben bei den beiden Blendenrastern wird innerhalb eines Bereiches gewählt, der 6 bis 100% des Abstandes der beiden Blendenraster beträgt.

Als Blendenraster werden zweckmässig fotografisch hergestellte Lochmasken auf der Basis von handelsüblichem fotografischem Material eingesetzt. Derartige Masken lassen sich leicht herstellen und besitzen darüber hinaus den Vorteil, dass sie von sehr geringer Dicke sind und damit Lichtreflexionen am Innenrand der transparenten Kreisscheiben weitgehend vermieden werden.

Bei dem Mikrodilutionsverfahren befinden sich die biologischen Proben in einer matrixähnlichen Anordnung von becherartigen Vertiefungen. (Mikrotiterplatten.) Vorteilhaft wird nun das erste Blendenraster direkt mit in die Probenhalterung integriert; d.h. das erste Blendenraster ist in diesem Fall Bestandteil der für die mikrobiologischen Struktur verwendeten Probenhalterung. Bei der für das Mikrodilutionsverfahren verwendeten Beleuchtungseinrichtung hat es sich ferner bewährt, wenn ein weiteres Blendenraster mit transparenten Kreisscheiben oberhalb der Probenhalterung, d.h. zwischen Probenhalter und Videokamera, angeordnet wird. Damit kann Störlicht ausgeblendet werden, das in der Probenhalterung, z.B. an den Rändern der becherartigen Vertiefungen durch Streuung oder Reflexionen entsteht.

Mit der Erfindung werden folgende Vorteile erzielt:

1. Im Vergleich zu den herkömmlichen Beleuchtungseinrichtungen konnte der Kontrast der mikrobiologischen Struktur gegenüber dem Untergrund erheblich verbessert werden. Bei den hier in Frage kommenden mikrobiologischen Strukturen handelt es sich in der Regel um eine Vielzahl von annähernd kreisförmigen Flecken, die in einem regelmässigen Flächenmuster auf einem transparenten Untergrund angeordnet sind (Agardilution oder um einen bzw. wenige relativ grosse kreisförmige transparente Bereiche auf lichtstreuendem Untergrund (Hemmhofmessung). Es liegt in der Natur dieser Strukturen, dass sich die Flecke optisch wegen des geringen Kontrastes nur wenig von ihrem Untergrund abheben, d.h. nur relativ schwach ausgeprägte Konturen besitzen. Durch die neue Beleuchtungseinrichtung werden die Kontraste deutlich angehoben, so dass die bildanalytische Auswertung wesentlich erleichtert und in vielen Fällen überhaupt erst ermöglicht wird. Die bildanalytische Auswertung umfasst die Erkennung der Objekte, ihrer Positionen und ihrer geometrischen und optischen Vermessung.

2. Das konstruktive Grundprinzip der Beleuchtungseinrichtung ist für alle mikrobiologischen Untersuchungsmethoden dasselbe, so dass abgesehen von leicht anzubringenden Zusatzeinrichtungen immer die gleiche Apparatur verwendet wird und keine aufwendigen Umbauten erforderlich sind.

3. Ein wesentlicher konstruktiver Vorteil besteht darin, dass die Beleuchtungseinrichtung raumsparend aufgebaut werden kann, weil das Blendensystem und die Lichtquelle in sehr geringem Abstand hintereinander angeordnet werden können und demzufolge nur eine geringe Bauhöhe erforderlich ist. Dadurch wird vor allem der nachträgliche Einbau in bereits vorhandene Apparaturen erleichtert.

4. Bei Anwendung der fotografischen Lochmaskentechnik lassen sich die Blendenraster mit der gewünschten Präzision auch kostengünstig herstellen.

Im folgenden werden Ausführungsbeispiele der Erfindung anhand von Zeichnungen näher erläutert. Es zeigen:

Fig. 1 eine Draufsicht der für das Agardilutionsverfahren verwendeten Probenhalterung,

Fig. 2 die wesentlichen Elemente der Beleuchtungseinrichtung beim Agardilutionsverfahren (Seitenansicht),

Fig. 3 ein Blendenraster in Draufsicht,

Fig. 4 die wesentlichen Elemente der Beleuchtungseinrichtung beim Mikrodilutionsverfahren (Seitenansicht),

Fig. 5 eine Ausführung der Beleuchtungseinrichtung für das Agardilutionsverfahren,

Fig. 6 eine Ausführung der Beleuchtungseinrichtung beim Mikrodilutionsverfahren und

Fig. 7 die wesentlichen Elemente der für die Hemmhofmessung und Koloniezählung verwendeten Beleuchtungseinrichtung (Seitenansicht).

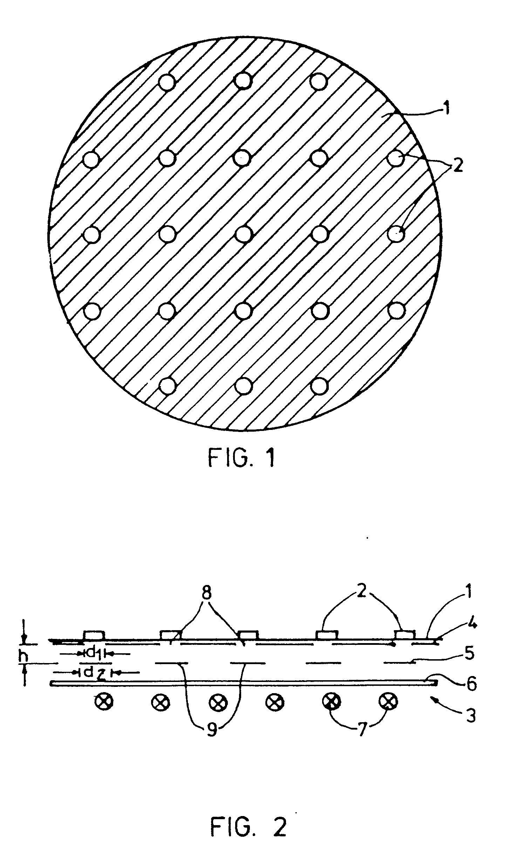

Gemäss Figur 1 sind für das Agardilutionsverfahren auf einem in einer Petrischale befindlichen Nährboden 1 einundzwanzig Impfpositionen in einem regelmässigen gitterförmigen Muster aufgebracht. An diesen 21 Positionen kann Bakterienwachstum auftreten. Es entsteht dann ein kreisförmiger Hof auf den Impfpositionen. Da die Bakterienarten im allgemeinen von Position zu Position verschieden sind und alle Bakterien durch eine einheitlich in den Nährboden eingearbeitete Antibiotikumskonzentration in ihrem Wachstum beeinflusst werden, ist an einigen Impfpositionen Bakterienwachstum zu beobachten, während an anderen Positionen kein Wachstum auftritt. Die Platte mit dem Nährboden ist durchsichtig und wird von der Unterseite her mit indirektem Licht beleuchtet. Die dazu verwendete Beleuchtungseinrichtung ist in Figur 2 dargestellt.

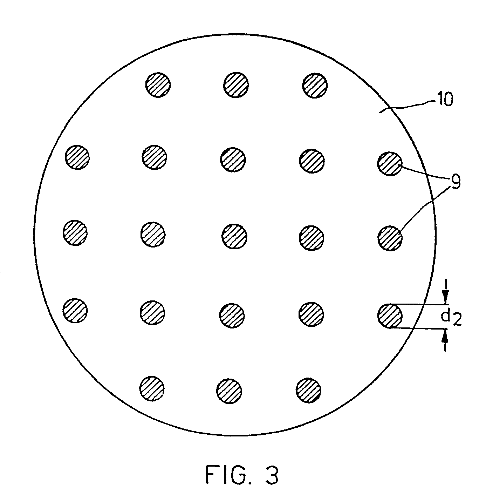

Sie besteht aus einer diffus strahlenden flächenförmigen Lichtquelle 3 und einem darüber angeordneten Blendensystem mit einem ersten Blendenraster 4 und einem zweiten Blendenraster 5. Der diffuse Flächenstrahler 3 besteht seinerseits aus einer Mattscheibe 6, die von unten durch eine Vielzahl von Glühlampen 7 beleuchtet wird. Die Glühlampen 7 befinden sich in einer Ebene und sind in gleichem Abstand voneinander in X- und Y-Richtung angeordnet. Das erste Blendenraster 4 besteht aus einer Vielzahl von transparenten Kreisscheiben 8 auf einem absorbierenden (lichtundurchlässigen) Untergrund und das zweite Blendenraster 5 aus einer Vielzahl von absorbierenden (lichtundurchlässigen) Kreisscheiben 9 auf einem transparenten Untergrund. Die beiden Blendenraster 4 und 5 sind also optisch komplementär. Das zweite Blendenraster 5 ist noch einmal in Figur 3 verdeutlicht. Man erkennt die im regelmässigen Abstand in zwei zueinander senkrechten Richtungen angeordneten schwarzen Kreisscheiben 9 auf dem transparenten Untergrund 10. Bei dem komplementären ersten Raster 4 sind die Kreisscheiben 8 transparent, während der Untergrund schwarz ist. Das erste Raster stellt also abgesehen von den geometrischen Kenngrössen, ein fotografisches Negativ des zweiten Blendenrasters dar. Der Durchmesser $d_2$ der lichtundurchlässigen Kreisscheiben 9 ist etwas grösser als der Durchmesser $d_1$ der transparenten Kreisscheiben 8. Ausserdem sind die beiden Blendenraster relativ zueinander derart angeordnet, dass die lichtundurchlässigen Kreisscheiben 9 des zweiten Blendenrasters konzentrisch zu den transparenten Kreisscheiben 8 des ersten Blendenrasters sind. Man erkennt aus Figur 2, dass sich die Kreisscheiben der beiden Blendenraster in der Parallelprojektion überlappen. Die transparenten Kreisscheiben 8 des ersten Blendenrasters 4 sind ihrerseits als konzentrische Kreisblenden den Impfpositionen 2 auf den Nährboden 1 zugeordnet. Die Impfpositionen 2 sind hier gleichbedeutend mit den Objektpositionen der mikrobiologischen Struktur. Der Abstand h der beiden Blendenraster 4 und 5 wird so gewählt, dass in der Objektebene eine weitestgehend ortsunabhängige konstante Beleuchtungsstärke herrscht. Diese Bedingung kann auf einfache Weise empirisch dadurch gefunden werden, dass man eine Mattscheibe anstelle der Probenhalterung in die Objektebene bringt, die sich dicht oberhalb des Blendenrasters 4 befindet.

Bezüglich der Dimensionierung der Blendenraster (geometrische Kenngrössen) gelten in der Praxis folgende Bedingungen:

$$0,2\,h \leqq d_1 \leqq d_2 \leqq 2\,h \text{ und } 0,05\,h \leqq d_2 - d_1 \leqq h$$

Die Herstellung der Blendenraster 4 und 5 erfolgt in einfacher Weise durch Belichtung von fotografischem Filmmaterial, das mit einer Lochmaske abgedeckt wurde. Das dazu komplementäre Blendenraster erhält man, wenn ein fotografisches Negativ der ersten Filmmaske angefertigt wird. Die fotografisch hergestellten Blendenraster haben den Vorteil, dass sie gegenüber Lochblenden aus Metall nur eine sehr geringe Dicke aufweisen.

Die Wirkungsweise der Beleuchtungseinrichtung gemäss Figur 2 beruht darauf, dass alle Objektpositionen 2 von der Unterseite her schräg beleuchtet werden (indirekte Beleuchtung) und direkt von der diffusen Lichtquelle 3 kommende Strahlen ausgeblendet werden und daher nicht in die zur Abbildung und Auswertung der mikrobiologischen Struktur verwendeten Fernsehkamera gelangen. Zur Ausleuchtung der Impfpositionen 2 tragen nur diejenigen Lichtanteile bei, die von den Transparenzbereichen in den beiden Blendenrastern 4 und 5 durchgelassen werden. Dies sind in bezug auf die optische Achse nur die schräg einfallenden Lichtanteile aus den Winkelbereichen der diffusen Beleuchtung. Dadurch wird die geforderte Schräglichtbeleuchtung aller möglichen Wachstumspositionen des Impfrasters auf dem Nährboden erreicht. Ferner wird jede Impfposition auf dem Nährboden aufgrund der konzentrischen Anordnung der zugehörigen Transparenzkreisscheibe des Blendenrasters 4 und der entsprechenden konzentrischen optisch undurchlässigen Kreisscheibe des Blendenrasters 5 von allen Seiten gleichmässig mit schräg einfallendem Licht beleuchtet. Die gesamte, aus den Blendenrastern 4 und 5 sowie der diffusen Flächenlichtquelle 3 bestehende Beleuchtungseinrichtung kann raumsparend mit wenigen Zentimetern Bauhöhe (5 bis 10 cm) realisiert werden.

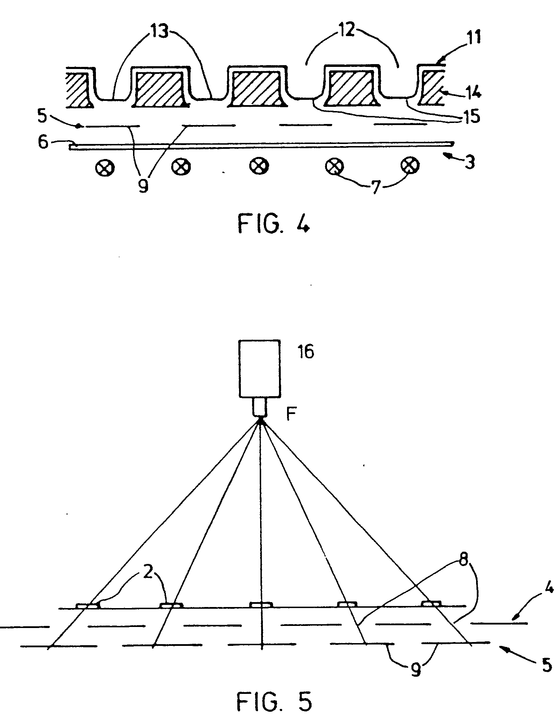

Beim Mikrodilutionsverfahren werden sogenannte Mikrodilutionsplatten 11 verwendet (siehe Figur 4). Sie bestehen aus einer Matrixanordnung von meistens 8 × 12 becherartigen Vertiefungen 12 und sind aus einem transparenten Kunststoff gefertigt. Der Boden 13 der Vertiefungen 12 ist entweder halbkugelartig oder flach ausgebildet. In die Vertiefungen 13 werden Substanzen von verschiedenen Antibiotika in unterschiedlicher Menge eingefüllt. Entlang einer Reihe (einer Achter-Reihe oder einer Zwölfer-Reihe), wird das gleiche Antibiotikum in zunehmender Menge eingegeben. Anschliessend werden Bakterien in einer Nährflüssigkeit zugegeben.

Danach wird die Mikrodilutionsplatte 11 bei 37°C inkubiert, wobei Bakterienwachstum stattfinden kann. In denjenigen Vertiefungen 13, in denen Wachstum stattfindet, entsteht eine optische Trübung.

Die Mikrodilutionsplatte 11 wird in eine aus Metall gefertigte Aufnahmehalterung 14 eingelegt. Die Aufnahmehalterung 14 enthält Vertiefungen, in die die becherartigen Vertiefungen 13 der Mikrodilutionsplatte 11 hineinpassen. Dadurch wird die eingelegte Mikrodilutionsplatte 11 eindeutig mechanisch fixiert. An der Unterseite der Aufnahmehalterung 14 befinden sich kreisförmige Öffnungen 15, die den Bodenbereich der becherartigen Vertiefungen 13 für den Lichteinfall von der Unterseite freilassen. Die Öffnungen 15 in der Aufnahmehalterung 14 entsprechen in ihrer Wirkung den transparenten Kreisscheiben 8 des ersten Blendenrasters 4 in Figur 2. Das erste Blendenraster ist also in die Aufnahmehalterung 14 integriert. Die zu untersuchende mikrobiologische Struktur wird hier durch die Gesamtheit der in den Vertiefungen 13 befindlichen Objekte (Nährlösungen mit Bakterien) gebildet.

Im übrigen ist die Beleuchtungseinrichtung ähnlich aufgebaut wie beim Agardilutionsverfahren nach Figur 1 bis 3. Das zweite Blendenraster 5 besteht wiederum aus schwarzen Kreisscheiben 9 mit etwas grösserem Durchmesser als die kreisförmigen Öffnungen 15 und ist in kurzem Abstand (wenige Zentimeter) derart angeordnet, dass die schwarzen Kreisscheiben 9 konzentrisch zu den Öffnungen 15 liegen. Als Flächenlichtquelle dient wieder eine Mattscheibe 6, die von unten her durch eine Vielzahl von Glühlampen 7 beleuchtet wird. Das durch die Öffnungen 15 gebildete erste Blendenraster und das dazu komplementäre zweite Blendenraster 5 verhindern wie in der Anordnung nach Figur 2 einen direkten Lichteinfall in die Vertiefungen 13. Die Proben in den Vertiefungen 13 werden also nur von Lichtstrahlen getroffen, die von der Lichtquelle 3 unter einem schrägen Winkel einfallen. Betrachtet man die Mikrodilutionsplatte 11 von oben her, so treten die Vertiefungen mit Bakterienwachstum aufgrund der durch Trübung hervorgerufenen Lichtstreuung optisch stark hervor. Die Lichtstreuung wird mit Hilfe einer Videokamera quantitativ ausgewertet.

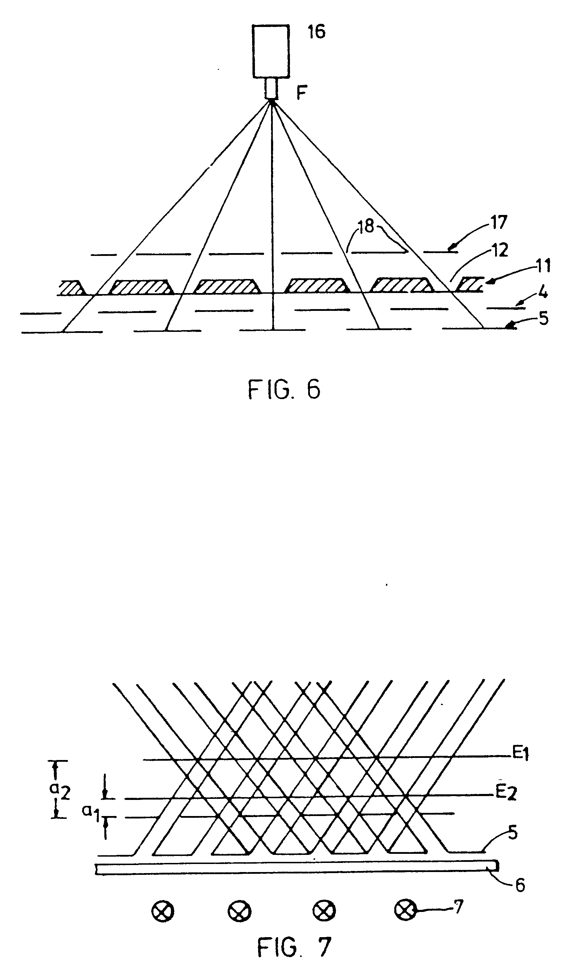

Anhand der Figuren 5 und 6 wird eine alternative Anordnung des Blendensystems mit einem verbesserten Schräglichteffekt beschrieben. Dabei entspricht Figur 5 dem Agardilutions- und Figur 6 dem Mikrodilutionsverfahren. Die Verbesserung besteht darin, dass die transparenten Kreisscheiben 8 des ersten Blendenrasters 4 und die absorbierenden Kreisscheiben 9 des zweiten Blendenrasters 5 nicht konzentrisch zueinander angeordnet, sondern seitlich gegeneinander verschoben sind. Die Anordnung wird so getroffen, dass die Mittelpunkte der Kreisscheiben der beiden Blendenraster auf der Verlängerung der Verbindungsgeraden von einem auf der optischen Achse liegenden Fluchtpunkt F zu den Objektpositionen (Impfpositionen) liegen. Dabei ist der Durchmesser der Transparenzscheiben 8 wie bei der Anordnung nach Figur 2 bis 4 wieder kleiner als der Durchmesser der undurchlässigen Scheiben 9 im Blendenraster 5. Sofern die oben angegebene Bedingung für alle perspektivischen Winkel, unter denen die Kamera 16 die Impfpositionen 2 sieht, eingehalten wird, ist eine optimale Schräglichtbeleuchtung gewährleistet, da der Durchmesser der undurchlässigen Scheiben 9 im Blendenraster 5 nur geringfügig grösser als der Durchmesser der transparenten Scheiben 8 zu sein braucht.

Bei der Herstellung der Blendenraster muss diesem perspektivischen Beleuchtungseffekt Rechnung getragen werden. Dies geschieht in einfacher Weise bei der oben erwähnten fotografischen Herstellung mittels Lochmasken dadurch, dass eine möglichst punktförmige Lichtquelle verwendet wird und der Abstand der Lichtquelle zu den fotografischen Folien genauso gross gewählt wird, wie der Abstand der Frontlinse des Kameraobjektivs von den Blendenrastern 4 und 5 bei der Apparatur nach Figur 5 (bzw. Figur 6) und bei der Belichtung der Abstand der Folien den gleichen Abstand einnehmen wie bei ihrem Einsatz in der Beleuchtungseinrichtung.

Gemäss Figur 6 wird der perspektivische Beleuchtungseffekt beim Mikrodilutionsverfahren ausgenutzt. Die Anordnung der Blendenraster 4 und 5 stimmt mit der in Figur 5 überein. Die Objektpositionen 2 entsprechen den Lösungen in den becherförmigen Vertiefungen 12 in der Probenhalterung 11. Um Störlicht auszublenden, das durch Streuung oder Reflexion in der Probenhalterung 11 hervorgerufen wird, ist oberhalb davon ein weiteres Blendenraster 17 mit transparenten Kreisscheiben 18 angeordnet. Für die Mittelpunkte der Kreisscheiben 18 gilt ebenfalls, dass sie auf den Verbindungsgeraden zwischen dem Mittelpunkt der Frontlinse der Videokamera 16 und den Objektpositionen liegen.

Anhand von Figur 7 wird die Anordnung zur Erzeugung einer indirekten Beleuchtung bei flächenhaften Bakterien-Wachstumsmustern beschrieben. Die Anordnung eignet sich insbesondere für die Hemmhofmessung auf runden Petrischalen, die Hemmhofmessung auf grossen rechteckigen Platten mit Hilfe eines zweidimensionalen Abtasttisches und die Koloniezählung. Bei der Hemmhofmessung müssen die Durchmesser von im allgemeinen kreisrunden Hemmhöfen erfasst werden. Zu diesem Zweck muss die Fläche in der Umgebung eines Hemmhofes bildanalytisch ausgewertet werden.

Bei der Koloniezählung entstehen nach Animpfung einer verdünnten Bakteriensuspension durch Inkubation bei 37°C aus einzelnen Bakterien jeweils Kolonien, deren Positionen und Anzahl auf der Fläche nicht bekannt sind. Es besteht nur die Aufgabe, die Anzahl der Kolonien zu ermitteln. Aus diesem Grund muss auch hier die ganze Fläche oder ein repräsentativer Teilausschnitt der Fläche bildanalytisch abgetastet werden.

Zur Beleuchtung dieser mikrobiologischen Strukturen wird wieder das Prinzip der komplementären Rasterblenden ausgenutzt. Gegenüber den Ausführungen nach Figur 1 bis 6 ist die Anordnung modifiziert. Bei den zuvor beschriebenen Methoden entsprach das Blendenrastermass dem Impfungsrastermass der biologischen Platte. Aus Figur 2 und Figur 4 ist z.B. ersichtlich, dass die transparenten Kreisscheiben 8 bzw. die Öffnungen 15 konkurrent zu den Objektpositionen sind. Zur Erzeugung einer indirekten Beleuchtung von mikrobiologischen Platten mit flächenhaften Objekten werden die Platten in einem grösseren bestimmten Abstand oberhalb des ersten Blendenrasters 4 angeordnet. Der optimale Abstand a lässt sich in folgender Weise experimentell ermitteln. Verfolgt man die von der Mattscheibe 6 ausgehenden Lichtbündel, die die Öffnungen in den beiden Blendenrastern 4 und 5 durchqueren können, so erkennt man, dass es mehrere Ebenen im Abstand $a_1$, $a_2$ oberhalb des Blendenrasters 4 gibt, die an nahezu jeder Stelle von einem der schräg einfallenden Lichtbündel getroffen werden. Bei Fortschreiten entlang der Schnittlinie dieser Ebenen wechseln Bereiche ab, die unter gleichem Winkel von schräg nach links oder schräg nach rechts verlaufenden Lichtbündeln geschnitten werden. Wichtig ist, dass hierbei in bezug auf die Platten normale gleiche Winkelbeträge in der Schräglichtbeleuchtung auftreten. Der Rechts-/Links-Wechsel in der Schräglichtbeleuchtung spielt jedoch für den Streulichtprozess an den Bakterienwachstumsbereichen keine Rolle.

Die Betrachtung von parallelen Strahlenbündeln gemäss Figur 7 stellt eine grobe Vereinfachung dar. In Wirklichkeit haben alle ausgeblendeten Lichtbündel eine erhebliche Divergenz. Diese wirkt sich im Sinne einer Vergleichmässigung der in den Ebenen $e_1$ und $e_2$ einfallenden Lichtintensitäten günstig aus. Die Lage der optimalen Ebene (oder der Ebenen) in der die Fluktuationen der einfallenden Lichtintensität minimal sind, lässt sich wie erwähnt, am besten experimentell feststellen. Zu diesem Zweck bringt man eine Streulichtscheibe im Abstand a an und vergewissert sich, dass an dieser Stelle minimale Fluktuationen der Streulichtintensität vorhanden sind.

Wie oben schon erläutert wurde, gibt es bei flächenhaften mikrobiologischen Strukturen keinen festen Zusammenhang zwischen den Blendenrastermassen und den Dimensionen der mikrobiologischen Struktur. Da sich auch bei optimalem Abstand a zwischen der Ebene des Messobjektes und dem ersten Blendenraster 4 eine geringfügige Restwelligkeit der Beleuchtungsstärke nicht vermeiden lässt, ist es günstig, wenn das Blendenrastermass klein im Vergleich zu den Dimensionen der erwarteten Objektstrukturen gewählt wird. Die Dimensionen der Objektstrukturen entsprechen in diesen Fällen dem Durchmesser der Hemmhöfe bzw. der Koloniegrösse.

Insgesamt ist deutlich geworden, dass das Prinzip der komplementären Blendenraster – abgesehen von methodenspezifischen Modifizierungen – universell zur Ausleuchtung mikrobiologischer Strukturen herangezogen werden kann, die durch Abtastung mit einer Videokamera bezüglich Lage (Lokalisierung) und Grösse (Durchmesserbestimmung) und optischen Eigenschaften (Dichte, Farbe u.a.) vollautomatisch analysiert werden. Durch die beschriebenen, apparatetechnisch mit relativ geringem Aufwand verbundenen Massnahmen, wird in allen Fällen der Kontrast der mikrobiologischen Strukturen gegenüber dem Untergrund deutlich angehoben. Dabei wird gleichzeitig erreicht, dass die Beleuchtungsstärke innerhalb der gesamten Objektfläche nahezu konstant ist und dass die Bauhöhe der Anordnung gering bleibt.

**Patentansprüche**

1. Beleuchtungseinrichtung für die optische, insbesondere bildanalytische Auswertung einer mikrobiologischen Struktur mit einer diffus strahlenden, flächenhaften Lichtquelle und einem zwischen der mikrobiologischen Struktur und der Lichtquelle angeordneten Blendensystem, dadurch gekennzeichnet, dass das Blendensystem durch zwei hintereinandergeschaltete, im Abstand befindliche, optisch komplementäre Blendenraster (4, 5) gebildet wird, dass das erste Blendenraster (4) aus einer Vielzahl von transparenten, periodisch aufeinanderfolgenden Kreisscheiben (8) auf einem lichtundurchlässigen Untergrund und das zweite Blendenraster (5) aus einer Vielzahl von lichtundurchlässigen, periodisch aufeinanderfolgenden Kreisscheiben (9) auf transparentem Untergrund besteht, und dass die lichtundurchlässigen Kreisscheiben (9) in ihrem Durchmesser grösser sind als die transparenten Kreisscheiben (8), so dass sich die Kreisscheiben der beiden Blendenraster (4 und 5) in der Projektion überlappen.

2. Beleuchtungseinrichtung nach Anspruch 1, dadurch gekennzeichnet, dass zur Untersuchung einer aus einer Vielzahl von Einzelobjekten bestehenden mikrobiologischen Struktur die Mittelpunkte der Kreisscheiben (8 und 9) der beiden Blendenraster (4 und 5) auf der Verlängerung der Verbindungsgeraden von einem auf der optischen Achse liegenden Fluchtpunkt F zu den Objektpositionen (2) liegen.

3. Beleuchtungseinrichtung nach Anspruch 1, dadurch gekennzeichnet dass zur Untersuchung einer aus relativ grossen, flächenhaften Objekten bestehenden mikrobiologischen Struktur die transparenten Kreisscheiben (8) des ersten Blendenrasters (4) im wesentlichen konzentrisch in Parallelprojektion zu den undurchlässigen Kreisscheiben (9) des zweiten Blendenrasters (5) angeordnet sind.

4. Beleuchtungseinrichtung nach Ansprüchen 1 bis 3, dadurch gekennzeichnet, dass der Abstand h der beiden Blendenraster (4, 5) und der Abstand zwischen dem ersten Blendenraster (4) und der Objektebene so gewählt werden, dass in der Objektebene eine weitgehend ortsunabhängige konstante Beleuchtungsstärke herrscht.

5. Beleuchtungseinrichtung nach Ansprüchen 1 bis 3, dadurch gekennzeichnet, dass der Abstand h zwischen den beiden Blendenrastern (4, 5) 0,5 bis 5 mal so gross ist, wie der Durchmesser der transparenten Kreisscheiben (8) des ersten Blendenrasters (4).

6. Beleuchtungseinrichtung nach Ansprüchen 1 bis 5, dadurch gekennzeichnet, dass die Differenz des Durchmessers der Kreisscheiben bei den beiden Blendenrastern (4, 5) 5% bis 100% des Abstandes der beiden Blendenraster beträgt.

7. Beleuchtungseinrichtung nach Ansprüchen 1 bis 6, dadurch gekennzeichnet, dass die Blendenraster (4, 5) aus fotografisch hergestellten Masken auf der Basis von handelsüblichem fotografischem Material bestehen.

8. Beleuchtungseinrichtung nach Ansprüchen 1 bis 7, dadurch gekennzeichnet, dass das erste Blendenraster (4) Bestandteil der für die mikrobiologische Struktur verwendeten Probenhalterung (14) ist.

9. Beleuchtungseinrichtung nach Ansprüchen 8, dadurch gekennzeichnet, dass oberhalb der Probenhalterung (14) ein weiteres Blendenraster (17) mit einer oder mehreren transparenten Kreisscheiben (18) zur Ausblendung von Störlicht angeordnet ist, das von der Probenhalterung (14) hervorgerufen wird.

## Claims

1. Illumination system for optical, in particular image analysis evaluation of a microbiological structure with a diffusely radiating, plane light source and an aperture system arranged between the microbiological structure and the light source, characterised in that the aperture system is formed by two optically complementary aperture screens (4, 5), one behind the other and at a distance from one another, in that the first aperture screen (4) consists in a plurality of transparent circular discs (8) in periodic sequence upon an opaque base and the second aperture screen (5) consists in a plurality of opaque circular discs (9) in periodic sequence on a transparent base, and in that the opaque circular discs (9) have a larger diameter than the transparent circular discs (8), as a result of which the circular discs of the two aperture screens (4 and 5) overlap in projection.

2. Illumination system according to claim 1, characterised in that for examining a microbiological structure consisting of a plurality of individual objects the centres of circular discs (8 and 9) of the two aperture screens (4 and 5) are located on the extension of the straight connecting line from a vanishing point F located on the optical axis to the object positions (2).

3. Illumination system according to claim 1, characterised in that for examining a microbiological structure consisting of relatively large, plane objects the transparent circular discs (8) of the first aperture screen (4) are arranged in substantially concentric manner in parallel projection to the opaque circular discs (9) of the second aperture screen (5).

4. Illumination system according to claim 3, characterised in that the distance h between the two aperture screens (4, 5) and the distance between the first aperture screen (4) and the object plane are selected in such a way as to bring about a largely position-independent constant intensity of illumination in the object plane.

5. Illumination system according to claims 1 to 3, characterised in that the distance h between the two aperture screens (4, 5) is 0.5 to 5 times as large as the diameter of the transparent circular discs (8) of the first aperture screen (4).

6. Illumination system according to claims 1 to 5, characterised in that the difference between the diameters of the circular discs of the two aperture screens (4, 5) amounts to 5% to 100% of the distance between the two aperture screens.

7. Illumination system according to claims 1 to 6, characterised in that the aperture screens (4, 5) consist of photographically produced masks based on commercially conventional photographic material.

8. Illumination system according to claims 1 to 7, characterised in that the first aperture screen (4) is part of the specimen holder (14) for the microbiological structure.

9. Illumination system according to claim 8, characterised in that above the specimen holder (14) a further aperture screen (17) is arranged with one or several transparent circular discs (18) for cutting out unwanted light caused by the specimen holder (14).

## Revendications

1. Dispositif d'éclairage pour l'évaluation optique, notamment par analyse d'image, d'une structure microbiologique comportant une source lumineuse plane à rayonnement diffus et un système de diaphragme disposé entre la structure microbiologique et la source lumineuse, caractérisé en ce que le système de diaphragme est formé de deux grilles de diaphragme (4, 5) optiquement complémentaires, montées à distance l'une derrière l'autre, en ce que la première grille (4) de diaphragme est constituée d'un certain nombre de disques circulaires (8) transparents se succédant périodiquement, sur un fond opaque, et la seconde grille de diaphragme (5) est constituée d'un certain nombre de disques circulaires (9) opaques, se succédant périodiquement, sur un fond transparent, et en ce que les disques circulaires opaques (9) ont un diamètre plus grand que celui des disques circulaires (8) transparents, en sorte que les disques circulaires des deux grilles (4 et 5) de diaphragme se chevauchent en projection.

2. Dispositif d'éclairage suivant la revendication 1, caractérisé en ce que pour l'examen d'une structure microbiologique constituée d'un certain nombres d'objets individuels, les centres des disques circulaires (8 et 9) des deux grilles (4 et 5) de diaphragmes sont situés sur le prolongement des droites joignant un centre de perspective (F) se trouvant sur l'axe optique aux positions d'objets (2).

3. Dispositif d'éclairage suivant la revendication 1, caractérisé en ce que pour l'examen d'une structure microbiologique constituée d'objets plans relativement grands, les disques circulaires transparents (8) de la première grille (4) de diaphragme ont une disposition principalement concentrique en projection parallèle par rapport aux disques opaques circulaires (9) de la seconde grille (5) de diaphragme.

4. Dispositif d'éclairage suivant la revendication 3, caractérisé en ce que la distance (h) des deux grilles de diaphragme (4, 5) et la distance entre la première grille (4) de diaphragme et le plan objet sont choisies de manière qu'un éclairement constant largement indépendant du lieu règne dans le plan objet.

5. Dispositif d'éclairage suivant les revendications 1 à 3, caractérisé en ce que la distance (h) entre les deux grilles de diaphragme (4, 5) représente 0,5 à 5 fois la valeur du diamètre des disques circulaires transparents (8) de la première grille (4) de diaphragme.

6. Dispositif d'éclairage suivant les revendications 1 à 5, caractérisé en ce que la différence de diamètre des disques circulaires dans le cas des deux grilles de diaphragme (4, 5) représente 5 à 100 % de la distance des deux grilles de diaphragme.

7. Dispositif d'éclairage suivant les revendications 1 à 6, caractérisé en ce que les grilles de diaphragme (4, 5) sont constituées de masques réalisés par photographie, à base d'un support photographique du commerce.

8. Dispositif d'éclairage suivant les revendications 1 à 7, caractérisé en ce que la première grille de diaphragme (4) est un constituant du porte-échantillon (14) utilisé pour la structure microbiologique.

9. Dispositif d'éclairage suivant la revendication 8, caractérisé en ce qu'une autre grille de diaphragme (17) comportant un ou plusieurs disques circulaires transparents (18) est disposée au-dessus du porte-échantillon (14) pour supprimer la lumière parasite qui est engendrée par le porte-échantillon (14).

FIG. 1

FIG. 2

FIG. 3

FIG. 4

FIG. 5

FIG. 6

FIG. 7